(19) **Europäisches Patentamt**
**European Patent Office**
**Office européen des brevets**

(11) Veröffentlichungsnummer: **0 452 807 B1**

(12) **EUROPÄISCHE PATENTSCHRIFT**

(45) Veröffentlichungstag der Patentschrift: **08.03.95**

(51) Int. Cl.6: **A61F 2/08**

(21) Anmeldenummer: **91105746.1**

(22) Anmeldetag: **11.04.91**

(54) **Resorbierbare Implantat-Kordel.**

(30) Priorität: **20.04.90 DE 4012602**

(43) Veröffentlichungstag der Anmeldung:
**23.10.91 Patentblatt 91/43**

(45) Bekanntmachung des Hinweises auf die
Patenterteilung:
**08.03.95 Patentblatt 95/10**

(84) Benannte Vertragsstaaten:
**DE FR GB IT**

(56) Entgegenhaltungen:
**EP-A- 0 041 111**
**EP-A- 0 312 494**
**WO-A-88/06872**
**WO-A-90/12550**
**GB-A- 1 256 579**

**PATENT ABSTRACTS OF JAPAN vol. 10, no.
055 (M-458)15. Oktober 1985**

(73) Patentinhaber: **ETHICON INC.**
**Route 22**
**Somerville,**
**New Jersey 08876-0151 (US)**

(72) Erfinder: **Hinsch, Bernhard, Dr.**
**Travestrasse 1**
**W-2000 Norderstedt (DE)**
Erfinder: **Walther, Christian, Dr.**
**Dorfstr. 35**
**W-2358 Kattendorf (DE)**

(74) Vertreter: **UEXKÜLL & STOLBERG Patentanwälte**
**Beselerstrasse 4**
**D-22607 Hamburg (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

**Beschreibung**

Die Erfindung betrifft eine resorbierbare Implantat-Kordel bestehend aus einer Seele aus Fäden oder Filamenten aus resorbierbarem Material, die von einem porösen oder durchlässigen Mantel aus resorbierbarem Material umschlossen ist.

Derartige resorbierbare Implantat-Kordeln, die beispielsweise für Augmentationsplastiken bei frischen und veralteten Kreuzbandrupturen, zur Stabilisierung der Symphysensprengung, zur Fixierung des Trochanter major nach Prothesenimplantation, zur Fixation nach einer Acromio-Clavicular-Sprengung und als Sternumverschluß eingesetzt werden, sind beispielsweise aus der EP 0 239 775 und der WO 88/06872 bekannt. Der Mantel gemäß der WO 88/06872 kann schlauchartig und mit einer netzartigen Struktur ausgebildet sein. Der Oberbegriff des Patentanspruchs 1 geht von einer resorbierbaren Implantat-Kordel gemäß WO-A-8806872 aus.

Diese bekannten resorbierbaren Prothesen haben zwar eine gewisse Reißfestigkeit, jedoch ist deren in-vivo-Reißkraftverlust verhältnismäßig groß und insbesondere nicht einstellbar. Die resorbierbare Prothese gemäß WO 88/06872, die auch als Bänderersatz dient und einen mehrschichtigen Aufbau hat, ist ungeeignet, weil dieser mehrschichtige Aufbau zur Lastabsorption durch Einbringung von Dehnungselementen erreicht wird, jedoch nicht zur Erhöhung der spezifischen Reißfestigkeit führt. Die einzelnen Schichten können verschiedene Resorptionszeiten haben, was durch verschieden resorbierbare Materialien bzw. durch Beschichtung dieser Materialien erreicht wird, jedoch werden mit diesen Implantaten nicht Masse-Resorptionen zu einem genau bestimmten Zeitpunkt erreicht und insbesondere ist der für die betreffende medizinische Indikation wichtigere Reißkraftabfall nicht steuerbar.

Aufgabe der Erfindung ist es, eine resorbierbare Implantat-Kordel der eingangs erwähnten Art vorzuschlagen, bei der einmal hohe Reißfestigkeiten und zum anderen ein einstellbarer und insbesondere geringer in-vivo-Reißkraftverlust erreicht wird.

Diese Aufgabe wird gelöst durch eine resorbierbare Implantat-Kordel mit einer Seele aus Fäden oder Filamenten aus resorbierbarem Material, die von einem porösen oder durchlässigen Mantel aus resorbierbarem Material umschlossen ist, der aus einem schlauchartigen Geflecht besteht, und wobei die Implantat-Kordel dadurch gekennzeichnet ist, daß der Mantel von einer weiteren Hülle aus resorbierbarem Material umschlossen ist, die aus einem schlauchartigen Geflecht besteht, daß das Geflecht des Mantels einen Flechtwinkel $\alpha$ im Bereich von 50 bis 89° hat, während der Flechtwinkel $\beta$ der Hülle in einem Bereich von 20 bis 80° liegt, daß die Flechtdichte $A_M$ des Mantels in einem Bereich von 1 000 bis 100 000 Überschneidungen/cm$^3$ und die Flechtdichte $A_H$ der Hülle in einem Bereich von 50 000 bis 1 000 000 Überschneidungen/cm$^3$ liegt, und daß die Implantat-Kordel durch Strecken der Implantat-Kordel bei Temperaturen von 60 bis 180°C um 10 bis 80% und anschließendes Abkühlen erhältlich ist.

Besonders bevorzugte Ausführungsformen sind in den Unteransprüchen aufgeführt.

Überraschenderweise hat sich gezeigt, daß man durch den erfindungsgemäßen Aufbau aus mindestens drei Schichten, nämlich einer Seele, die gefacht, geflochten oder gezwirnt sein kann, einem aufgeflochtenen Mantel und einer weiteren aufgeflochtenen Hülle aus resorbierbarem Material eine möglichst hohe Reißfestigkeit erhält. Man kann zwar bei diesen Kordeln, bei denen alle Schichten eine offene Struktur haben müssen, das Verhältnis von Seele zu Mantel so auswählen, daß eine möglichst hohe Reißfestigkeit resultiert; man kann auch bei einer Seele/Mantel-Struktur durch den Flechtwinkel des Mantels die Reißfestigkeit erhöhen, jedoch zeigen diese bekannten Kordeln mit zwei-schichtigem Aufbau in Abhängigkeit von der Flechtdichte einen sehr unterschiedlichen in-vivo-Reißkraftabfall bei gleichem bzw. sehr ähnlichen Molekulargewichtsabbau.

Bei den oben erwähnten Indikationen sind hohe Reißfestigkeiten von Vorteil, jedoch wird besonders für Augmentationsplastiken bei Freuzbandrupturen auch ein langsamerer in-vivo-Reißkraftverlust, also eine sehr hohe Halbwertszeit des Reißkraftabbaus, von mehr als 35 Tagen gefordert, wenngleich zur Fixation von AC-Sprengungen auch Materialien mit einer geringeren Halbwertszeit von 22 bis 25 Tage ausreichend sind. Überraschenderweise läßt sich nun mit der erfindungsgemäßen Implantat-Kordel neben der offenen Struktur und der hohen spezifischen Festigkeit ein einstellbares in-vivo-Abbauprofil erreichen, das Halbwertzeiten der Festigkeiten bis zu 60 Tagen und mehr bei bestimmten resorbierbaren Materialien ermöglicht.

Die erwähnte offene Struktur ist von Vorteil, um das Einwachsen von Bindegewebe und das Einsprossen von Knochen in das Implantat zu ermöglichen.

Wesentlich ist bei den erfindungsgemäßen Implantaten, daß deren Verhalten dem von nativen Bändern und Sehnen angenähert ist und im Linearzug ähnliche Steifigkeiten und ähnliche viskoelastische Eigenschaften bei sehr viel höherer Reißkraft aufweisen, wie dieses beispielsweise in der Fachliteratur von CLAES, "Biochemische Eigenschaften humaner Bänder"; Rehm, Unfallchirurgie 11(1985) 228-229 und E.R. Noyes, D.L. Butler et. al in "Biomechanical Analysis of Human Ligaments Grafts used in Knee-Ligament Repairs and Reconstructions" The Journal of Bone and Joint Surgery, Band 66-A, Nr. 3, 344-352/194 gefordert wird.

Besonders vorteilhaft ist es, daß sich bei den erfindungsgemäßen Kordeln die in-vivo-Halbwertszeit von beispielsweise PDS-Kordeln bei gleichem Ausgangsmaterial von beispielsweise einem 5 x 12 dpf-PDS-Multifilamentgarn zwischen etwa 14 bis 54 Tagen einstellen läßt und somit das für die verschiedenen Einsatzgebiete dieser chirurgischen Implantate jeweils geeignete optimale Reißkraftabbauprofil auswählbar ist.

Resorbierbare Polymere mit guter physiologischer Verträglichkeit sind an sich bekannt. Typische Vertreter sind die Poly-p-dioxanone, die unter der Bezeichnung "PDS" im Handel sind und chemisch gesehen durch ringöffnende Polymerisation hergestellte aliphatische Polyester des p-Dioxanon sind, sich zu Monofilamenten extrudieren lassen und bei 105 bis 115°C schmelzen; sie sind in "Ethicon OP Forum", Heft 108 (1981) und in J. Pediatr. Ophthalmol. 13, 360-364 (1976) beschrieben.

Zu einer anderen Gruppe resorbierbarer Materialien gehören die Polyglactine, die Copolymere aus Glykolid und Lactid sind und sich ebenfalls zu Fäden extrudieren lassen, die unter der Bezeichnung "Vicryl" (Wz) im Markt erhältlich und im einzelnen beispielsweise in "Ethcon OP Forum", Heft 96 (1978) und in J. Pediatr. Ophthalmol. 13, 360, 1976 beschrieben sind. Zu dieser Gruppe gehören auch Lactid-Glykolid-Blockcopolymerisate gemäß DE-OS 28 49 785. Diese Polyglactine haben je nach Polymerisationsgrad einen Schmelzpunkt von 180 bis 225°C.

Je nach Art des resorbieren Materials, der Herstellungsbedingungen und auch je nach Polymerisationsgrad ist die Resorbierbarkeit verschieden. Sie liegt beispielsweise bei den höher schmelzenden Polyglactinen in einem Bereich von 60 bis 80 Tagen und bei den niedriger schmelzen Poly-p-dioxanonen in einem Bereich von 200 Tagen. Durch entsprechende Mischung der das Implantat bildenden Monofilamenten oder Fasern kann man die Resorptionsdauer des gesamten Implantats, also die Schnelligkeit, mit welcher das Implantat abgebaut werden kann, steuern.

Besonders bevorzugt ist eine Mischung von Faseren des Poly-p-dioxanons mit solchen des Polyglactins in einem Verhältnis von 5:1 bis 1:15 und insbesondere in einem Verhältnis von 1:3.

Im folgenden soll die Erfindung anhand von Beispielen in Zusammenhang mit den beiliegenden Figuren näher erläutert werden; es zeigen:

Figur 1: eine schematische und teilweise gebrochene Seitenansicht der erfindungsgemäßen resorbierbaren Implantat-Kordel;

Figur 2: einen Querschnitt durch die Kordel gemäß Figur 1.

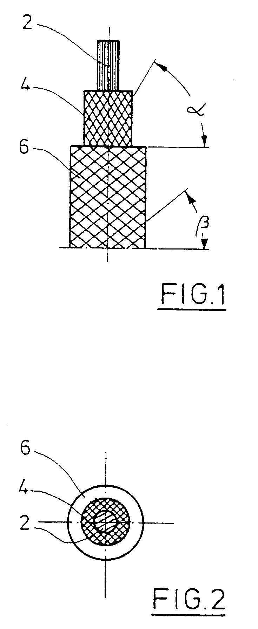

Die in den Figuren 1 und 2 gezeigte Kordel besteht aus einer Seele aus gefachten, geflochtenen oder gezwirnten Fäden oder Filamenten aus resorbierbarem Material, wobei vorzugsweise längsgerichtete parallel nebeneinander liegende Fäden oder Filamente bevorzugt werden.

Der aufgeflochtene Mantel oder Flechtmantel 4 aus resorbierbarem Material besteht aus einem schlauchartigen Geflecht mit einem Flechtwinkel $\alpha$ im Bereich von 50 bis 89° und vorzugsweise von 65 bis 88°. Die Flechtdichte des Mantels 4 kann in einem Bereich von 1 000 bis 100 000 Überschneidungen/cm$^3$ liegen.

Die Hülle 6 besteht aus einem aufgeflochtenen resorbierbaren Material mit einem Flechtwinkel in einem Bereich von 20 bis 80 und vorzugsweise von 30 bis 72° und einer Flechtdichte von 50 000 bis 1 000 000 und vorzugsweise von 80 000 bis 500 000 Überschneidungen/cm$^3$.

Das Gewichtsverhältnis von dem aus Seele 2 und Mantel 4 bestehenden Kern der Implantat-Kordel zur Hülle 6, beträgt vorzugsweise 2,5:1 bis 25:1.

Die wesentlichen Parameter der Implantat-Kordel sind einmal der Flechtwinkel und zum anderen die Flechtdichte.

Die Berechnung des Flechtwinkels $\alpha$ für das Geflecht des Mantels bzw. des Flechtwinkels $\beta$ für das Geflecht der Hülle ergibt sich durch folgende Formel:

$$\tan g\ \alpha = \frac{\dfrac{2,54\ [cm]}{B}}{\dfrac{\phi\ [cm]\ *\ \pi}{a}} = \frac{2,54\ *\ a}{B\ *\ \phi\ *\ \pi}$$

in welcher B die Flechtungen je Zoll bzw. je 2,54 cm und a die Anzahl der Klöppel, also der Spulen am Flechtkopf und $\phi$ der Durchmesser der Kordel bedeuten.

Zur Berechnung der Flechtdichte, also der Überschneidungen je cm$^3$, ergibt sich die Flechtdichte A, bzw. die der Hülle $A_H$ und die des Mantels $A_M$ aus der folgenden Formel:

$$A = \frac{B * a * 1.000.000 \text{ [cm]} * D \text{ [g/cm}^3\text{]}}{\text{dtex der Hülle bzw. des Mantels [g]} * 2,54 \text{ [cm]}}$$

wobei B die Flechtungen je Zoll bzw. je 2,54 cm in Fadenrichtung und a wiederum die Anzahl der Carrier bzw. der Spulen im Flechtkopf und D die Dichte des Fadenmaterials in g/cm³ bedeuten, die im vorliegenden Fall etwa bei 1,38 g/cm³ liegt. Der Wert von dtex der Hülle bzw. des Mantels in g entspricht einer Fadenlänge von 10 000 m Faden und bei Angaben von "den" dem Gewicht in g von 9 000 m Faden.

Es wurden folgende resorbierbare Implantat-Kordeln hergestellt.

Beispiel 1:

Zur Herstellung einer Kordel mit einem Durchmesser von 1,5 mm aus einem 5 x 12 dpf-Polydioxanon-Multifilamentgarns, was einem 60 den Garn entspricht, wurde die folgende Flechstruktur hergestellt, wobei die Angabe 5 x 12 dpf sich auf "denier per fiber" bezieht und mit dem Faktor 1,11 in dtex je Fiber umrechenbar ist.

Die Seele bestand aus einem gefachten Multifilamentgarn;
(6 x (9 x 60) = 3240 den).

Der Mantel bestand aus einer mit 16 Klöppeln geflochteten Struktur mit etwa 22 Flechtungen je Zoll bzw. 2,54 cm und ca. 8640 den; (16 x (9 x 60) den).

Die Flechtdichte $A_M$ betrug etwa 20 000 Überschneidungen/cm³.

Der Flechtwinkel $\alpha$ betrug 75,7°.

Die Hülle bestand aus einem mit 16 Klöppeln geflochtenen 60 den Multifilamentgarn mit insgesamt 2880 den (16 x (3 x 60) und mit etwa 55 Flechtungen je Zoll bzw. 2,54 cm. Der Flechtwinkel $\beta$ der Hülle lag bei 57,5°.

Die Flechtdichte $A_H$ betrug etwa 149 000 Überschneidungen/cm³.

Die Reißfestigkeit lag bei 328 N/mm².

Die Reißkraft lag bei etwa 600 N.

Die Halbwertszeit in-vivo betrug etwa 54 Tage.

Beispiel 2

Es wurde eine Kordel mit 3 mm Durchmesser aus 5 x 12 dpf-Polydioxanon-Multifilamentgarnen (60 den Garn) mit folgenden Einzelheiten hergestellt:

| | |
|---|---|
| Seele: | 7 (36 x 60), gefacht, d.h. 15120 den |
| Mantel: | 16 Klöppel x (36 x 60) mit etwa 9 Flechtungen je Zoll bzw. 2,54 cm, d.h. 34560 den |
| Flechtdichte $A_M$: | 2000 Überschneidungen/cm³ |
| Flechtwinkel $\alpha$: | 78,2°, |
| Hülle: | 16 Klöppel x (4 x 60) mit etwa 60 Flechtungen je Zoll bzw. 2,54 cm, d.h. 3840 den |
| Flechtwinkel $\beta$: | = 35,7° |
| Flechtdichte $A_H$ | etwa 122 000 Überschneidungen/cm³, |
| Reißfestigkeit: | etwa 350 N/mm², |
| Reißkraft: | etwa 2 500 N |
| Halbwertszeit in-vivo: | etwa 50 Tage. |

Beispiel 3:

Es wurde eine Kordel mit einem Durchmesser von 1,2 mm aus 5 x 12 dpf-Multifilamentgarn aus Polydioxanon hergestellt, wobei Seele, Mantel und Hülle die folgenden Abmessungen hatten:

| | |
|---|---|
| Seele: | 7 (7 x 60), gefacht, (2940 den), |
| Mantel: | 16 Klöppel x (7 x 60), (6720 den), |
| Flechtung: | 17 je Zoll bzw, 2,4 cm, |
| Flechtdichte $A_M$: | 2000/cm³, |
| Flechtwinkel $\alpha$: | = 81,0°, |
| Hülle: | 16 Klöppel x (2 x 60), (1920 den), |

4

| | | |
|---|---|---|
| Flechtung: | 60 je Zoll bzw. 2,54 cm, |
| Flechtwinkel $\beta$: | 60,9°, |
| Flechtdichte $A_H$: | etwa 258 000 Überschneidungen/cm$^3$, |
| Reißkraft: | etwa 450 N, |
| Reißfestigkeit: | etwa 398 N mm$^2$, |
| Halbwertszeit in vivo: | etwa 60 Tage |

Beispiel 4:

Es wurde eine Kordel mit einem Durchmesser von 2,0 mm aus 5 x 12 dpf Multifilamentgarn aus Polydioxanon hergestellt, wobei die einzelnen Elemente die folgende Spezifikation erfüllten:

| | |
|---|---|
| Seele: | 7 (19 x 60), gefacht, (7980 den), |
| Mantel: | 16 Klöppel x (19 x 60), (18240 den), |
| Flechtung: | 8,5 Flechtungen je Zoll bzw. 2,54 cm, |
| Flechtdichte $A_M$: | 3650/cm$_3$, |
| Flechtwinkel $\alpha$: | 82,5°, |
| Hülle: | 16 Klöppel x (4 x 60), |
| Flechtung: | 64 Flechtungen je Zoll bzw. 2,54 cm, |
| Flechtwinkel $\beta$: | 45,3°, |
| Flechtdichte $A_H$: | etwa 128 000 Überschneidungen je cm$^3$, |
| Reißfestigkeit: | etwa 350 N/mm$^2$, |
| Reißkraft: | etwa 1 130 N |
| Halbwertszeit in vivo: | etwa 50 Tage. |

Um den Einfluß der Flechtdichte in Flechtungen je Zoll oder je 2,54 cm bzw. in Überschneidungen/cm$^3$ auf die in-vivo-Halbwertszeit bzw. den Reißkraftabfall und die Reißfestigkeit von PDS-Kordeln zu zeigen, wurden verschiedene Kordeln nach dem Stand der Technik bestehend aus Seele und Umhüllung (Versuche 1 bis 8) bzw. mit einer Umhüllung und einer gezwirnten Seele (Versuch 9) und einer erfindungsgemäßen Kordel mit dreischichtigem Aufbau (Versuch 10) untersucht, wobei eine erfindungsgemäße Kordel gemäß Versuch 10 mit 58 Flechtungen je Zoll bzw. je 2,54 cm und einem Mantel mit etwa 26 Flechtungen verwendet wurde.

| Versuch | Flechtungen/2,54 cm | Durchmesser [mm] | in-vivo-Halbwertzeit (Tage) | Reißkraft [N] | Reißfestigfestigkeit [N/mm$^2$] | Überschneidungen/cm$^3$ |
|---|---|---|---|---|---|---|
| 1 | 22 | 1,2 | 14,4 | 436 | 386 | 22.400 |
| 2 | 26±3 | 0,8 | 16 | 220 | 438 | 52.970 |
| 3 | 32-33 | 1,0 | 18 | 310 | 395 | 44.140 |
| 4 | 22 | 1,5 | 21,4 | 664 | 375 | 15.000 |
| 5 | 25 | 2,0 | 22 | 600 | 190 | 12.730 |
| 6 | 36±2 | 1,5 | 26 | 430 | 243 | 25.930 |
| 7 | 44 | 1,0 | 31 | 210 | 267 | 72.400 |
| 8 | 47±2 | 1,2 | 31 | 300 | 265 | 48.300 |
| 9 | 45-50 | 1,7 | 34 | 570 | 251 | 90.000 |
| 10 | 55 | 1,5 | 54 | 580 | 328 | 147.000 |

Die Werte dieser Tabelle zeigen den Einfluß der Flechtdichte auf die in-vivo-Halbwertszeit, also den Reißkraftverlust auf 50%, gemessen in Tagen bei verschiedenen Kordeln mit einem Durchmesser von 0,8 bis 2,0 mm und der erfindungsgemäßen Kordel.

Die Tabelle zeigt, daß bei hoher spezifischer Reißfestigkeit von über 3000 N/mm$^2$ der in-vivo-Reißkraftabfall, also die Halbwertszeit, relativ schnell abläuft und in einem Bereich von 14 bis 22 Tagen liegt, während bei der erfindungsgemäßen Kordel gemäß Versuch 10 bei einer Halbwertszeit von 54 Tagen die Reißfestigkeit bei etwa 328 N/mm$^2$ liegt.

Bei einem Vergleich gleicher Stärken ergeben sich deutliche Unterschiede. Kordeln mit einem Durchmesser von 1,0 mm, nämlich gemäß Versuch 3 und 7, besitzen bei Halbwertszeiten von 18 und 31 Tagen Reißfestigkeiten von 395 und 267 N/mm$^2$. Die Kordeln mit einem Durchmesser von 1,2 mm gemäß Versuch 1 und 8 zeigen bei Halbwertszeiten von 14,4 und 31 Tagen Festigkeiten von 386 und 265 N/mm$^2$.

Die Kordeln mit einem Durchmesser von 1,5 mm gemäß Versuch 4, 6 und 10 zeigen bei Halbwertszeiten von 21,4 Tagen bzw. 26 Tagen und ca. 54 Tagen Reißfestigkeiten von 375 bzw. 243 bzw. 328 N/mm², wobei die erfindungsgemäße Kordel gemäß Versuch 10 neben einer ausreichend hohen Reißfestigkeit auch eine extrem lange in-vivo-Haltbarkeit zeigte.

Zur Bestimmung der biomechanischen Eigenschaften wie Steifigkeit, Reißkraft und Reißdehnung, die vom Querschnitt der Implantate und auch vom Implantationsort abhängig sind, wurden die Eigenschaften nativer Bänder mit den Eigenschaften von erfindungsgemäßen Implantaten verglichen.

Die viskoelastischen Eigenschaften von nativen Bändern gegenüber dem erfindungsgemäßen Implantat gemäß Beispiel 1 ergeben sich aus der folgenden Aufstellung, wobei die Kraftrückgewinnung in Prozent je Minuten nach CLAES (a.a.o.) gemessen wurde.

Tabelle II

| Untersuchtes Band | Kraftrückgewinnung in %/min |
|---|---|
| Seitenband, nativ | 4 bis 5% |
| Kreuzband, nativ | 3,7 (± 0,8%) |
| erfindungsgemäße Kordel gemäß Beispiel 1 | 4 bis 5% |

Die biomechanischen Eigenschaften von nativen Bändern gegenüber erfindungsgemäßen PDS-Kordeln ergeben sich aus der folgenden Tabelle III.

Tabelle III

| | Kollateralbänder* | Kreuzbänder* | | PDS-Kordel mit ⌀ von | | |
|---|---|---|---|---|---|---|
| | | anterior | posterior | 1,5 mm | 2,00 mm | 3,0 mm |
| Reißkraft[N] | 384-654 | 350-1.730 | 571-1.745 | 600 | 900 | >2.000 |
| Reißdehnung [%] | 18-23 | 30-44 | 283 | 38 | 36 | 39 |
| Steifigkeit [N/mm] | 47-94 | 129-182 | 183 | 80 | 90 | ca. 170 |

* nach L. CLAES

Wenngleich die Fäden oder Filamente vor der Verarbeitung zur Kordel in der Regel einem Heißstreck-Verfahren unterworfen werden, wird vorzugsweise die fertige Kordel einem Heißstreck-Verfahren unterworfen und anschließend getempert. Bei dem Heißstreck-Verfahren wird die Kordel über beheizte Galletten oder durch einen beheizten Kanal mit einer Lufttemperatur von 60 bis 180°C und vorzugsweise 65 bis 90°C geführt und einem Streckverhältnis von 10 bis 80 und vorzugsweise 15 bis 70% unterworfen. Die unter Spannung auf Rahmen gewickelten Kordeln werden dann in einem trockenen Stickstoffstrom bei Temperaturen zwischen 60 und 105°C und vorzugsweise 70 bis 95°C 6 bis 48 Stunden nachbehandelt.

Bei den Kordeln gemäß Beispiel 1 bis 4 erfolgte das Heiß-Strecken bei 75°C bei einem Streckverhältnis von 40%, während beim nachfolgenden Tempern 24 Stunden lang bei Temperaturen von 85°C gearbeitet wurde.

Je nach dem eingesetzten resorbierbaren Material kann das Tempern im Verlaufe von etwa 6 bis 48 Stunden erfolgen.

**Patentansprüche**

**1.** Resorbierbare Implantat-Kordel mit einer Seele (2) aus Fäden oder Filamenten aus resorbierbarem Material, die von einem porösen oder durchlässigen Mantel (4) aus resorbierbarem Material umschlossen ist, der aus einem schlauchartigen Geflecht besteht, **dadurch gekennzeichnet**, daß der Mantel (4) von einer weiteren Hülle (6) aus resorbierbarem Material umschlossen ist, die aus einem schlauchartigen Geflecht besteht, daß das Geflecht des Mantels (4) einen Flechtwinkel $\alpha$ im Bereich von 50 bis 89° hat, während der Flechtwinkel $\beta$ der Hülle (6) in einem Bereich von 20 bis 80° liegt, daß die Flechtdichte $A_M$ des Mantels (4) in einem Bereich von 1 000 bis 100 000 Überschneidungen/cm³ und die Flechtdichte $A_H$ der Hülle (6) in einem Bereich von 50 000 bis 1 000 000 Überschneidungen/cm³ liegt, und daß die Implantat-Kordel durch Strecken der Implantat-Kordel bei Temperaturen von 60 bis 180°C um 10 bis 80% und anschließendes Abkühlen erhältlich ist.

2. Resorbierbare Implantat-Kordel nach Anspruch 1, **dadurch gekennzeichnet**, daß die Seele (2) aus längsgerichteten parallel nebeneinander liegenden Fäden oder Filamenten aus resorbierbarem Material besteht.

3. Resorbierbare Implantat-Kordel nach Anspruch 1 und 2, **dadurch gekennzeichnet**, daß das schlauchartige Geflecht des Mantels (4) einen Flechtwinkel im Bereich von 65 bis 88° hat.

4. Resorbierbare Implantat-Kordel nach Anspruch 1 bis 3, **dadurch gekennzeichnet**, daß der Flechtwinkel der Hülle (6) in einem Bereich von 30 bis 72° liegt.

5. Resorbierbare Implantat-Kordel nach Anspruch 1 bis 4, **dadurch gekennzeichnet**, daß die Flechtdichte des Mantels (4) in einem Bereich von 1 500 bis 80 000 Überschneidungen/cm$^3$ liegt.

6. Resorbierbare Implantat-Kordel nach Anspruch 1 bis 5, **dadurch gekennzeichnet**, daß die Flechtdichte der Hülle (6) in einem Bereich von 80 000 bis 500 000 Überschneidungen/cm$^3$ liegt.

7. Resorbiere Implantat-Kordel nach Anspruch 1 bis 6**, dadurch gekennzeichnet**, daß das Gewichtsverhältnis von dem aus Seele (2) und Mantel (4) bestehenden Kern zur Hülle (6) in einem Bereich von 2,5:1 bis 25:1 liegt.

8. Resorbierbare Implantat-Kordel nach Anspruch 1 bis 7, **dadurch gekennzeichnet**, daß die Resorbierbarkeit der den Mantel (4) bildenden Fäden oder Filamente von der die Hülle (6) bildenden Fäden oder Filamente verschieden ist.

9. Resorbierbare Implantat-Kordel nach Anspruch 1 bis 8, **dadurch gekennzeichnet**, daß sie durch nach der Streckung auszuführendes 6- bis 48-stündiges Tempern unter Spannung bei Temperaturen zwischen 60 und 180°C erhältlich ist.

10. Resorbierbare Implantat-Kordel nach Anspruch 1, **dadurch gekennzeichnet**, daß sie durch Streckung um 40% bei etwa 75°C und anschließendes 24-stündiges Tempern bei 85°C unter trockenem Stickstoffstrom erhältlich ist.

## Claims

1. Absorbable implant cord having a central strand (2) which is composed of threads or filaments of absorbable material and is surrounded by a porous or permeable casing (4) which is composed of absorbable material and which consists of a tubular braiding, characterized in that the casing (4) is surrounded by another sheath (6) which is composed of absorbable material and which consists of a tubular braiding, in that the braiding of the casing (4) has a braiding angle $\alpha$ in the range from 50 to 89°, while the braiding angle $\beta$ of the sheath (6) is in a range from 20 to 80°, in that the braiding density $A_M$ of the casing (4) is in a range from 1,000 to 100,000 crossovers/cm$^3$ and the braiding density $A_H$ of the sheath (6) is in a range from 50,000 to 1,000,000 crossovers/cm$^3$, and in that the implant cord can be obtained by stretching the implant cord at temperatures from 60 to 180°C by 10 to 80% and subsequently cooling.

2. Absorbable implant cord according to Claim 1, characterized in that the central strand (2) consists of threads or filaments which are composed of absorbable material and are directed lengthwise and lie parallel side by side.

3. Absorbable implant cord according to Claim 1 and 2, characterized in that the tubular braiding of the casing (4) has a braiding angle in the range from 65 to 88°.

4. Absorbable implant cord according to Claim 1 to 3, characterized in that the braiding angle of the sheath (6) is in a range from 30 to 72°.

**5.** Absorbable implant cord according to Claim 1 to 4, characterized in that the braiding density of the casing (4) is in a range from 1,500 to 80,000 crossovers/cm$^3$.

**6.** Absorbable implant cord according to Claim 1 to 5, characterized in that the braiding density of the sheath (6) is in a range from 80,000 to 500,000 crossovers/cm$^3$.

**7.** Absorbable implant cord according to Claim 1 to 6, characterized in that the ratio by weight of the core consisting of the central strand (2) and casing (4) to the sheath (6) is in a range from 2.5:1 to 25:1.

**8.** Absorbable implant cord according to Claim 1 to 7, characterized in that the absorbability of the threads or filaments forming the casing (4) is different from that of the threads or filaments forming the sheath (6).

**9.** Absorbable implant cord according to Claim 1 to 8, characterized in that it can be obtained by heat treatment under tension at temperatures between 60 and 180°C to be carried out for 6 to 48 hours after the stretching.

**10.** Absorbable implant cord according to Claim 1, characterized in that it can be obtained by stretching by 40% at about 75°C and subsequent heat treatment at 85°C under a stream of dry nitrogen for 24 hours.

**Revendications**

**1.** Cordon implantable résorbable comportant une âme (2) formée de fils ou de filaments réalisés en un matériau résorbable et qui est entourée par une gaine poreuse ou perméable (4) formée d'un matériau résorbable et qui est constituée par une tresse en forme de tuyau, caractérisé en ce que la gaine (4) est entourée par une autre enveloppe (6) formée d'un matériau résorbable et qui est constituée par une tresse en forme de tuyau, que la tresse de la gaine (4) possède un angle de tressage $\alpha$ dans la gamme de 50 à 89°, tandis que l'angle de tressage $\beta$ de l'enveloppe (6) se situe dans une gamme de 20 à 80°, que la densité de tressage A$_M$ de la gaine (4) se situe dans une gamme de 1000 à 100 000 croisements/cm$^3$ et que la densité de tressage A$_H$ de la gaine (6) est située dans une gamme de 50 000 à 1 000 000 croisements/cm$^3$, et que le cordon implantable peut être obtenu par un étirage de 10 à 80 % du cordon implantable à des températures de 60 à 180°C et refroidissement ultérieur.

**2.** Cordon implantable résorbable selon la revendication 1, caractérisé en ce que l'âme (2) est constituée par des fils ou des filaments longitudinaux formés d'un matériau résorbable et disposés parallèlement côte-à-côte.

**3.** Cordon implantable résorbable selon les revendications 1 et 2, caractérisé en ce que la tresse en forme de tuyau de la gaine (4) possède un angle de tressage dans la gamme de 65 à 88°.

**4.** Cordon implantable résorbable selon les revendications 1 à 3, caractérisé en ce que l'angle de tressage de l'enveloppe (6) est situé dans une gamme de 30 à 72°.

**5.** Cordon implantable résorbable selon les revendications 1 à 4, caractérisé en ce que la densité de tressage de la gaine (4) est située dans une gamme de 1500 à 80 000 croisements/cm$^3$.

**6.** Cordon implantable résorbable selon les revendications 1 à 5, cractérisé en ce que la densité de tressage de l'enveloppe (6) est située dans une gamme de 80 000 à 500 000 croisements/cm$^3$.

**7.** Cordon implantable résorbable selon les revendications 1 à 6, caractérisé en ce que le rapport du poids du noyau constitué par l'âme (2) de la gaine (4), au poids de l'enveloppe (6) est situé dans une gamme allant de 2,5:1 à 25:1.

**8.** Cordon implantable résorbable selon les revendications 1 à 7, caractérisé en ce que la capacité de résorption des fils ou filaments constituant la gaine (4) est différente de la capacité de résorption des fils ou filaments constituant l'enveloppe (6).

9. Cordon implantable résorbable selon les revendications 1 à 8, caractérisé en ce qu'il peut être obtenu au moyen d'un recuit d'une durée de 6 à 48 heures, devant être exécuté après l'étirage et sous contrainte à des températures comprises entre 60 et 180°C.

10. Cordon implantable résorbable selon la revendication 1, caractérisé en ce qu'on peut l'obtenir par étirage de 40 % à environ 75°C, puis recuit pendant 24 heures à 85°C dans un courant d'azote sec.

FIG.1

FIG.2